# NEUE EUROPÄISCHE PATENTSCHRIFT

(11) **EP 1 717 223 B2**
(45) Veröffentlichungstag und Bekanntmachung des Hinweises auf die Entscheidung über den Einspruch: **04.01.2023**
(45) Hinweis auf die Patenterteilung: 20.07.2016
(21) Anmeldenummer: 06118257.2
(22) Anmeldetag: 16.12.2003
(51) Int. Cl.: C07C 263/10, C07C 263/20, C07C 265/14

(54) **Verfahren zur Reinigung von Isocyanaten**
Method for the purification of isocyanates
Procédé de purification d'isocyanates

(30) Priorität: 19.12.2002 DE 10260092
(43) Veröffentlichungstag der Anmeldung: 02.11.2006
(62) Teilanmeldung aus: 03789289.0
(73) Patentinhaber: BASF SE, 67056 Ludwigshafen am Rhein (DE)
(72) Erfinder: Wölfert, Andreas, 74906, Bad Rappenau (DE); Pallasch, Hans-Jürgen, 67169, Kallstadt (DE); Stroefer, Eckhard, 68163, Mannheim (DE); Blankertz, Heinrich-Josef, 67147, Forst (DE)
(74) Vertreter: Ellwanger, Arndt

(56) Entgegenhaltungen:
- US-A- 2 680 127
- US-A- 3 140 305
- US-A- 3 410 888
- US-A- 3 471 543
- US-A- 4 216 063
- US-A- 5 962 728

## Beschreibung

Die vorliegende Erfindung betrifft ein Verfahren zur Reinigung eines Produktstroms aus der Isocyanatsynthese.

Unter Isocyanaten werden hier Verbindungen mit 1, 2 oder mehr Isocyanatgruppen verstanden (Mono-, Di- oder Polyisocyanate), bevorzugt Diisocyanate.

Das erfindungsgemäße Verfahren eignet sich für alle gängigen (cyclo)aliphatischen und aromatischen Isocyanate, oder ein Gemisch aus zwei oder mehr solcher Isocyanate. Bevorzugt werden Diisocyanate, beispielsweise monomeres Methylen-di(phenylisocyanat (MDI), Toluylendiisocyanat (TDI), R,S-1-Phenylethylisocyanat, 1-Methyl-3-phenylpropylisocyanat, Naphtyldiisocyanat (NDI), n-Pentylisocyanat, 6-Methyl-2-heptanisocyanat, Cyclopentylisocyanat, Hexamethylendiisocyanat (HDI), Isophorondiisocyanat (IPDI), 2,4- und 2,6-Di-isocyanato-methyl-cyclohexan (H₆TDI) und deren Isomerengemische, o-, m- oder p-Xylendiisocyanat (XDI), Di-isocyanato-cyclohexan (t-CHDI), Di-(isocyanato-cyclohexyl)-methan (H₁₂MDI), Tetramethyl-m-xylylendiisocyanat (m-TMXDI), 1,3-Bis(isocyanatomethyl)cyclohexan (H₆XDI), Di-isocyanato-cyclohexan (t-CHDI), 1,6-Di-isocyanato-2,2,4,4-tetramethylhexan, 1,6-Di-isocyanato-2,2,4-trimethylhexan und deren Gemische (TMDI).

Besonders bevorzugt wird das Verfahren zur Reinigung von TDI, Monomer-MDI, HDI, IPDI, H6TDI, H12MDI, XDI, t-CHDI und NDI eingesetzt.

Es sind in der Literatur bereits verschiedene Verfahren zur Herstellung von Reinisocyanat beschrieben.

In US 3,410,888 wird ein Verfahren zur Isolierung und Reinigung von Isocyanaten beschrieben. Das Verfahren umfaßt die Schritte erstens der Reaktion eines entsprechenden Diamins mit Phosgen und der destillativen Abtrennung eines Teils des so hergestellten Isocyanats im Zuge der Lösungsmittelabtrennung, zweitens der Überführung des Destillationsrückstandes (Sumpfproduktes) in eine zweite Destillationseinrichtung (Gefäß), über dessen innere Oberfläche der Rückstand als dünner Film verteilt ist und dessen Temperatur und Druck ausreichend sind, um eine Verdampfung des Isocyanats zu bewirken, und drittens der Entnahme des Dampfes (Brüden) aus dieser zweiten Destillationseinrichtung, der im wesentlichen reich an Isocyanat ist.

Der Dampf wird kondensiert und ergibt das Isocyanat. Als mögliche Destillationseinrichtungen werden Kletterfilmverdampfer, Fallfilmverdampfer u.ä. genannt. Das gewählte Lösungsmittel in der Isocyanatsynthese hat üblicherweise einen niedrigeren Siedepunkt als das Isocyanat, bevorzugt mindestens 30°C niedriger. Bei einer kleineren Siedepunktsdifferenz wird allerdings in der Lösungsmittelabtrennung ein Teil des hergestellten Isocyanats zusammen mit dem Lösungsmittel abgetrennt. Daran schließt sich die Destillation des als Rückstand erhaltenen Rohisocyanats im Dünnschichtverdampfer an. Die teilweise Abtrennung des Isocyanats in der Lösungmittelabtrennung hat den Vorteil, dass unerwünschte Mittelsieder (ggf. gefärbte Verunreinigungen oder Komponenten, deren Siedepunkt zwischen dem des Isocyanats und dem Lösungsmittel liegt), in der Lösungsmittelabtrennung mit abgetrennt werden. Die Mischung aus dem teilweise abgetrennten Isocyanat und dem Lösungsmittel wird dann wieder als Einsatzstoffstrom der Lösungsmittelabtrennung zugeführt, oder sie wird in einer separaten Verdampfung oder fraktionierten Destillation zur Aufkonzentration des Isocyanats zugeführt. Letzteres wird dann als Feed in die Lösungsmittelabtrennung rezykliert.

Nachteilig an diesem Verfahren ist, daß durch die Verdampfung in zwei Schritten bei isomeren Isocyanaten, wie z.B. TDI oder MDI, durch die unterschiedliche Flüchtigkeit der Isomere Fraktionen unterschiedlicher Zusammensetzung erhalten werden. Die Reinheit der erhaltenen Isocyanate genügt zudem nicht den heutigen Anforderungen, da noch Leichtsieder enthalten sind. Ebenso geht über den hochsiedenden Rückstand Produkt verloren, da der Rückstand fließfähig sein muß, um aus dem Verdampfer herausgefördert werden zu können.

In der älteren deutschen Anmeldung mit dem Aktenzeichen 10245584.8 und dem Einreichetag 27.09.2002 wird ein Verfahren zur Herstellung von Isocyanaten beschrieben, wobei der Reaktionsaustrag in Form einer Suspension vorliegt, die das herzustellende Isocyanat als Flüssigkeit und Carbamoylchlorid als Feststoff enthält, in dem die Suspension in einem Schichtverdampfer aufgearbeitet wird. Dieser Schichtverdampfer besitzt bevorzugt keine bewegten Teile, wie beispielsweise ein Fallfilmverdampfer. Die Aufarbeitung kann auch in mehreren Druckstufen in zwei oder mehreren hintereinandergeschalteten Schichtverdampfern erfolgen, wobei der erste Schichtverdampfer bei einem Druck von 0,5 bis 25 bar arbeitet und der Druck des zweiten um 0,01 bis 1 bar geringer ist als der des ersten.

US 5,962,728 beschreibt die Verwendung eines Dünnschichtverdampfers in Verbindung mit einem Schaufeltrockner und einer Leichtsiederabtrennkolonne. Dabei wird das Rohisocyanat einem Dünnschichtverdampfer zugeführt. In dem Dünnschichtverdampfer wird das Reinisocyanat vom schwersiedenden, polymeren Teer getrennt, wobei die Trennung nicht vollständig ist, um den Teer genügend dünnflüssig zu halten. Der noch Reste des Wertproduktes enthaltende Teerstrom wird einem Schaufeltrockner zugeführt, in dem das Restisocyanat aus dem Teer abgedampft wird. Das dampfförmige Isocyanat, das noch leichtsiedende Verunreinigungen besitzt wird, abschließen einer destillativen Leichtsiederabtrennung unterzogen. Das Verfahren zeichnet sich dadurch aus, dass der Schaufeltrockner gleichzeitig eine Heiz- und eine Kühlzone besitzt.

Nachteilig an dem Verfahren ist, dass der die Anordnung verlassende Isocyanatstrom immer als Sumpfprodukt entnommen wird, und damit hohen thermischen Belastungen ausgesetzt ist, was zu Verlusten an NCO-Gruppen durch Oligomerisierung oder Polymerisierung des Diisocyanates führt, da solche Hochsieder nicht abgetrennt werden.

US 3,140,305 beschreibt die Verwendung eines horizontalen Dünnschichtverdampfers zur Rückgewinnung aromatischer Diisocyanate. Nachteilig ist an dem Verfahren, dass das rückgewonnene TDI noch leichtsiedende Verunreinigungen aufweist, was die direkte Verwendung des Reaktionsaustrages als Edukt in der Polyurethanherstellung erschwert. Zudem geht Wertprodukt aus dem hochsiedenden Rückstand verloren.

US 4,216,063 beschreibt die Rückgewinnung von Toluylendiisocyanat (TDI) in einem Dünnschichtverdampfer bei einer Wandtemperatur von 210°C bis 250°C und einem Druck von 1 mm Hg bis 50 mm Hg mit einer Mindestverweilzeit von 15 min. Nachteilig ist an dem Verfahren, dass das rückgewonnene TDI noch leichtsiedende Verunreinigungen aufweist, was die direkte Verwendung des Reaktionsaustrages als Edukt in der Polyurethanherstellung erschwert.

US 3471543 beschreibt Destillationseinheiten zur Aufreinigung von isocyanathaltigen Strömen eine Reihe von Dünnschichtverdampfern.

Aufgabe der Erfindung war es, ein Verfahren zur Herstellung von reinem Isocyanat durch Reinigung eines Rohisocyanatstromes bereitzustellen, bei der das gewünschte Reinisocyanat mit möglichst hoher Reinheit und hoher Ausbeute gewonnen werden kann, wobei gleichzeitig der apparatetechnische und energetische Aufwand möglichst gering gehalten werden soll.

Die Aufgabe wurde gelöst durch ein Verfahren zur Reinigung eines Produktstroms aus der Isocyanatsynthese, umfassend eine
- destillative Reinigung eines Rohisocyanatstromes (1) im Vakuum bei 1 bis 120 mbar und bei Temperaturen von 90 bis 170 °C in einen gasförmigen Brüden (3) und einen flüssigen Teilstrom (2),
- den nicht verdampfbaren Rückstand im Teilstrom (2) von dem Brüdenstrom (3) und/ oder von Stoffströmen, die den Brüdenstrom (3) zumindest teilweise enthalten, getrennt halten,
- Abtrennung von im flüssigen Teilstrom (2) enthaltenem Isocyanat von darin enthaltenem, nicht verdampfbaren Rückstand als isocyanathaltiger dampfförmiger Reststrom (4),
- destillatives Auftrennen des isocyanathaltigen dampfförmigen Reststroms (4) und des Brüdenstroms (3) in drei Einzelströme (5,6,7) mit unterschiedlichen Siedebereichen, wobei der am leichtesten siedende Strom (5) einen wesentlichen Teil des Leichtsiederanteils des Rohisocyanatstroms (1) enthält, der am schwersten siedende Strom (7) einen wesentlichen Teil des Hochsiederanteils des Rohisocyanatstroms (1) enthält, und der mittelsiedende Strom (6) im Wesentlichen Wertprodukt enthält,
wobei man die Abtrennung in einem Schaufeltrockner ohne Kühlzone und mit Austragsschnecke für den nichtverdampfbaren Rückstand (8) durchführt.

Die Erfindung wird üblicherweise angewendet in einem Verfahren zur Reinigung von Isocyanaten, in dem man
a) einen Strom (1), enthaltend Isocyanat, höher und niedrigersiedende Komponenten, sowie nicht verdampfbaren Rückstand, in einer mindestens eine theoretische Trennstufe enthaltenden Destillation in einen Teilstrom (2), der einen nicht verdampfbaren Rückstand und Isocyanat enthält, und in einen Isocyanat und Leichtsieder enthaltenden Brüdenstrom (3) auftrennt,
b) den nicht verdampfbaren Rückstand im Teilstrom (2) von dem Brüdenstrom (3) und/oder von Stoffströmen, die den Brüdenstrom (3) zumindest teilweise enthalten, getrennt hält,
c) aus dem Teilstrom (2) mindestens einen weiteren isocyanathaltigen Brüdenstrom (4) und einen im wesentlichen nicht verdampfbaren Rückstand enthaltenden Strom (8) auftrennt und
d) den oder die isocyanathaltigen Brüdenströme (4) und den Brüdenstrom (3) aus a) in drei Einzelströme (5, 6,7) mit unterschiedlichen Siedebereichen destillativ auftrennt, wobei der am leichtesteten siedende Strom (5) einen wesentlichen Teil des Leichtsiederanteils des Rohisocyanatstromes (1) enthält, der am schwersten siedende Strom (7) einen wesentlichen Teil des Hochsiederanteils des Rohisocyanatstromes (1) enthält, und der mittelsiedende Strom (6) im wesentlichen Wertprodukt enthält.

Für das erfindungsgemäße Verfahren ist es unerheblich, nach welchem Verfahren das eingesetzte Rohisocyanat erhalten worden ist, beispielsweise durch Phosgenierung eines Amins, durch Phosgenierung eines Aminhydrochlorids oder eines Carbamatsalzes, nach dem sog. Harnstoffverfahren, wie z.B. in EP 18 586, EP 566 925, EP 355 443 oder EP 568 782 beschrieben, oder durch Umsetzung des zugrundeliegenden Amins mit Dialkylcarbonaten, wie es beispielsweise in EP-A2 976 723 beschrieben ist. Bevorzugt wird das Isocyanat durch Phosgenierung hergestellt.

Die Herstellung der Isocyanate erfolgt üblicherweise durch Umsetzung des entsprechenden primären Amines oder dessen Hydrochlorids mit einem Überschuß an Phosgen. Dabei findet dieser Prozess üblicherweise in flüssiger Phase in einem Lösungsmittel oder in der Gasphase statt. Bevorzugt wird der Prozess in der Flüssigphase durchgeführt.

Üblicherweise wird als Lösungsmittel ein Lösungsmittel eingesetzt, das leichter siedet als das Isocyanat, z.B. Chlorbenzol, o- oder p-Dichlorbenzol, Trichlorbenzol, Chlortoluole, Chlorxylole, Chlorethylbenzol, Chlornaphthaline, Chlordiphenyle, Methylenchlorid, Perchlorethylen, Toluol, Xylole, Hexan, Dekahydronaphthalin, Diethylisophthalat (DEIP) und andere Carbonsäureester, wie sie beispielsweise in der US 5,136,086, Spalte 3, Zeilen 3 bis 18 aufgeführt sind, Tetrahydrofuran (THF), Dimethylformamid (DMF), Benzol und deren Gemische. Auch kann das hergestellte Isocyanat oder ein Stoffstrom des Verfahrens als Lösungsmittel verwendet werden. Besonders bevorzugt ist Chlorbenzol und Dichlorbenzol. Das inerte Lösungsmittel kann bevorzugt zu Beginn der Umsetzung dem Amin zugesetzt werden. Der Gehalt an Amin im Gemisch aus Amin und Lösungsmittel beträgt 1 bis 50 Massen-%, bevorzugt 3 bis 40 Massenprozent.

Es ist aber auch denkbar, das Amin in reiner Form einzusetzen, wenn die Phosgenierung in der Gasphase durchgeführt wird.

Das aus dem Reaktor austretende Umsetzungsgemisch (Reaktionsaustrag) liegt meist in Form einer Suspension vor. Diese Suspension enthält das herzustellende Isocyanat als Flüssigkeit und noch nicht zersetzte Carbamylchloride als Feststoffe. Gegebenenfalls enthält die aus dem Reaktor austretende Suspension weiterhin Aminhydrochloride und/oder Harnstoffe (R-NH-CO-NH-R) als Feststoffe.

Der Reaktionsaustrag enthält noch Reste des bei der Phosgenierung entstandenen Chlorwasserstoffs, das überschüssige Phosgen, das Lösungsmittel sowie Verunreinigungen und nicht verdampfbaren Rückstand polymerer Natur. Die Verunreinigungen und der polymere Rückstand sind während der Reaktion durch unvollständige Umsetzung oder unerwünschte Neben- oder Folgereaktionen entstanden. Dabei werden üblicherweise zunächst der Chlorwasserstoff und Phosgen in einem, oder mehreren Schritten aus dem Reaktionsaustrag entfernt. Anschließend wird das Lösungsmittel abdestilliert. Aus dem so erzeugten Rohisocyanat wird dann unter Anwendung des erfindungsgemäßen Verfahrens das Reinisocyanat hergestellt.

Alternativ kann das Isocyanat auch durch Phosgenierung in der Gasphase hergestellt werden. Dabei wird der Reaktionsaustrag, der das Isocyanat enthält, aus der Gasphase in einem inerten Lösungsmittel gequencht. Auch hier eignet sich das erfindungsgemäße Verfahren zur Herstellung des Reinisocyanates aus dem entsprechenden Rohisocyanat nach Abtrennung von Chlorwasserstoff, Phosgen und Lösungsmittel. Eventuell kann die Gasphasenphosgenierung auch ohne die Verwendung eines zusätzlichen Lösungsmittels erfolgen. Dann kann das erfindungsgemäße Verfahren auf den Rohisocyanatstrom bereits nach Abtrennung von Chlorwasserstoff und Phosgen angewendet werden.

Üblicherweise ergeben sich wirtschaftlich relevante Ausbeuteverluste während der Herstellung des Reinisocyanates aus dem Rohisocyanatstrom, die dadurch entstehen, dass der Wertstoff Reinisocyanat längere Verweilzeiten in Regionen höherer Temperatur aufweist. Durch die Temperaturbelastung kommt es zur Oligomerenbildung des Isocyanates (Isocyanurat, Carbodiimid, Urethdion etc.), was die Ausbeute herabsetzt. Das Problem der Ausbeuteverluste wird üblicherweise dadurch gelöst, dass die Reindestillation im Hochvakuum durchgeführt wird. Durch die Destillation im Hochvakuum wird das Temperaturniveau in der Destillationsvorrichtung herabgesetzt, was die Polymerisationsneigung verringert. Nachteilig bei diesem Vorgehen ist jedoch, dass die wegen des Hochvakuums geringen Dichten im Gasraum der Destillationseinrichtung zu großen Brüdenvolumenströmen führen. Große Brüdenvolumenströme erfordern dann große Kolonnendurchmesser, um fluidmechanisch sinnvolle Gasgeschwindigkeiten in der Destillationsvorrichtung einzuhalten. Die großen Kolonnendurchmesser sind mit unerwünscht hohen Investitionen verbunden.

Aus dem in die erfindungsgemäße Reinigung einzusetzenden Strom (1) (Rohisocyanat) ist nach der Herstellung des Isocyanats für den Fall der bevorzugten Herstellung durch Phosgenierung Chlorwasserstoff, Phosgen und Lösungsmittel im wesentlichen abgetrennt, so daß der Gehalt an Chlorwasserstoff und Phosgen jeweils unter 1000 ppm beträgt und der Lösungsmittelgehalt unter 1 Gew.-%, bevorzugt unter 0,5 Gew.-% und besonders bevorzugt unter 0,1 Gew.-% beträgt.

Der Rohisocyanatstrom (1) enthält neben dem als Wertprodukt zu gewinnenden Isocyanat üblicherweise 100 ppm bis 5 % leichter als das Isocyanat siedende Komponenten (Leichtsieder), 100 bis 5000 ppm höher als das Isocyanat siedende Komponenten (Hochsieder), deren Siedepunkt bei Normaldruck jedoch nicht mehr als 60 °C höher ist als der Siedepunkt des Isocyanates, und 1-8 Gew.-% nicht verdampfbaren Rückstand polymerer Natur, d.h., das Produkt pyrolisiert bevor es bei Normaldruck verdampft.

Erfindungsgemäß wird, zum Beispiel in Schritt a) eines Verfahrens zur Reinigung von Isocyanaten, der Rohisocyanatstrom (1) im Vakuum bei 1 bis 120 mbar, bevorzugt 1 - 100 mbar und bei Temperaturen von 90 bis 170, bevorzugt 100 bis 160 °C destillativ in mindestens einem theoretischen Trennboden aufgetrennt in einen gasförmigen Brüden-strom (3) und einen flüssigen Teilstrom (2). Diese Destillation erfolgt bevorzugt als einstufige Verdampfung. Sie kann beispielsweise aus einem Vorlagebehälter und einem Verdampfer erfolgen, die im Kreislauf betrieben werden. Falls gewünscht, kann dieser Verfahrensschritt auch in einer Kolonne oder in einem Teil einer Trennwandkolonne mit trennwirksamen Einbauten durch-geführt werden. In diesem Fall wird diese Kolonne oder der Teil der Trennwandkolonne nur mit einem Abtriebsteil mit Verdampfer und ohne Verstärkungsteil und ohne Rücklauf betrieben, der durch Kondensation der Brüden erzeugt werden würde, um der Anforderung b) gerecht zu werden, dass der nicht verdampfbare Rückstand im Teilstrom (2) von dem Brüdenstrom (3) und/oder von Stoffströmen, die den Brüdenstrom (3) zumindest teilweise enthalten, getrennt gehalten wird.

Als trennwirksame Einbauten kommen alle üblichen in Destillationskolonnen einsetzbaren Einbeuten in Frage. Bevorzugt werden Einbauten, die einen geringen Druckverlust aufweisen wie Füllkörper, strukturierte Packungen oder Dual-Flow-Böden. Besonders bevorzugt werden strukturierte Packungen.

Die Erzeugung der Gasphase kann durch den Betrieb eines Verdampfers erfolgen, bevorzugt wird ein Dünnschichtverdampfer, ein Kletterverdampfer, ein Fallfilmverdampfer, ein Langrohrverdampfer oder ein Zwangsumlaufentspannungsverdampfer verwendet. Besonders bevorzugt wird ein Zwangsumlaufentspannungsverdampfer oder ein ein Fallfilmverdampfer verwendet.

Die in Schritt a) entstehenden Brüden (3) bestehen im wesentlichen aus Isocyanat und Leichtsiedern. Allerdings werden durch den relativ großen Brüdenstrom auch noch nennenswert verdampfbare Hochsieder mitgestrippt. Ihr Gehalt liegt in der Regel unter 0,5%.

Der flüssige Teilstrom (2) enthält den aufkonzentrierten nicht verdampfbaren Rückstand und alle anderen Bestandteile des Rohisocyanatstromes (1), der Gehalt an Leichtsiedern beträgt in der Regel unter 1 Gew.-%, bevorzugt unter 0,5 Gew.-%.

Die Ströme (2) und (3) werden in der Regel im Gewichtsverhältnis 20:1 - 1:1 aufgeteilt, bevorzugt 10:1 - 1:1 und besonders bevorzugt 8:1 - 4:1.

Der flüssige Teilstrom (2) wird dann zur Rückgewinnung des darin enthaltenen Isocyanats, zum Beispiel in Schritt c) eines Verfahrens zur Reinigung von Isocyanaten, von darin enthaltenem, nicht verdampfbaren Rückstand getrennt. Dies erfolgt in einem Schaufeltrockner ohne Kühlzone und mit Austragsschnecke für den nichtverdampfbaren Rückstand (8). In diesem Verfahrensschritt wird mindestens ein vor allem isocyanathaltiger dampfförmigen Reststrom (4) erzeugt.

Der Schritt c) erfolgt in der Regel bei 80 - 320 °C, bevorzugt 100 - 300 °C und einem Druck von 0,1 - 40 mbar, bevorzugt 0,5 - 20 mbar.

Der verbleibende Rückstandsstrom (9) enthält in der Regel weniger als 2,5 Gew.-% Wertprodukt, bevorzugt weniger als 1,5 Gew.-% und besonders bevorzugt weniger als 0,5 Gew.-% und ist in der Regel entweder hochviskos oder fest und wird üblicherweise verbrannt oder deponiert.

Der Dampfstrom (4) oder falls mehrere Dampfströme (4) erzeugt werden, die Dampfströme (4), gegebenenfalls nach Kondensation, und der Brüdenstrom (3) werden dann gasförmig oder nach Kondensation, bevorzugt nach Kondensation, üblicherweise in einem Verfahren zur Reinigung von Isocyanaten in einem Schritt d) rektifikativ in einen leichter als das Reinisocyanat siedenden Strom (5), einen mittelsiedenden Strom (6), dessen Siedepunkt nahe, beispielsweise im Bereich von +/- 20 °C, bevorzugt +/- 10 °C, besonders bevorzugt +/- 5 C an dem des Reinisocyanates liegt, und einen schwerer als das Isocyanat siedenden Strom (7) aufgetrennt.

Die Auftrennung des isocyanathaltigen Brüdenstromes (4) und des Reststromes (3) findet in mindestens einer, bevorzugt 1 oder 2, besonders bevorzugt einer Destillationsvorrichtung statt.

Möglich ist zum Beispiel als erste Ausführungsform in einer ersten Destillationsvorrichtung d1) die Auftrennung des Brüdenstromes (4) und des Reststromes (3) in einen schwersiedenden Strom (7), der im wesentlichen Hochsieder enthält, und in einen weiteren Reststrom, der in einer weiteren Destillationsvorrichtung d2) in den leichtsiedenden Strom (5) und in den mittelsiedenden Reinisocyanatstrom (6) aufgetrennt wird.

Die Zufuhr zur ersten Destillationseinrichtung d1) kann dampfförmig oder nach vorheriger Kondensation flüssig erfolgen. Die Zufuhr erfolgt bevorzugt nach vorheriger Kondensation in flüssiger Phase.

Eine bevorzugte zweite Ausführung ist die Anwendung einer Destillationsvorrichtung d1) zur Auftrennung des Brüdenstrom (4) und des Reststromes (3) in einen leichsiedenden Strom (5) und in einen weiteren Restrom, der in einer Destillationsvorrichtung d2) in den schwersiedenden Strom (7) und in den mittelsiedenden Reinisocyanatstrom (6) aufgetrennt wird. Die Zufuhr zur ersten Destillationseinrichtung d1) kann dampfförmig oder nach vorheriger Kondensation flüssig erfolgen. Die Zufuhr erfolgt bevorzugt nach vorheriger Kondensation in der flüssigen Phase.

In einer dritten besonders bevorzugten Ausführung erfolgt die destillative Auftrennung des Reststromes (3) und des isocyanathaltigen Brüdenstromes (4) in einer Destillationskolonne mit einer Trennwand. Die Zufuhr kann dampfförmig oder nach vorheriger Kondensation flüssig erfolgen. Die Zufuhr der Brüdenstrome (4) erfolgt bevorzugt nach vorheriger Kondensation in der flüssigen Phase. Auf der Ablaufseite, die von der Zulaufseite durch die Trennwand abgegrenzt ist, wird das Reinisocyanat (6) entnommen. Am Kopf der Kolonne wird der leichtsiedende Strom (5) abgezogen. Am Sumpf wird der hochsiedende Strom (7) entnommen.

Die Destillationsvorrichtungen werden in der Regel bei 1 - 80 mbar betrieben und einer Sumpftemperatur von 100 - 240 °C. Sie weisen in der Regel jeweils 1 bis 50 theoretische Trennstufen auf und sind von an sich bekannter Bauart.

Bevorzugt werden die beiden Ströme (4) und (3) gemeinsam in einer Destillationsvorrichtung rektifikativ aufgetrennt. Die Auftrennung in die drei Ströme (5,6,7) erfolgt unter Verwendung von mindestens 2 bis 50 theoretischen Trennstufen, bevorzugt von mindestens 8 bis 30 theoretischen Trennstufen. Der Kopfdruck beträgt in der Regel 4 - 80 mbar und die Sumpftemperatur von 110 bis 240 °C. Der Zulauf der Ströme (4) erfolgt bevorzugt unterhalb des Stromes (3). Die Ströme (5) und (7) werden einer Verbrennung oder Deponierung zugeführt. Der Strom (5) wird bevorzugt der Verbrennung zugeführt.

Als trennwirksame Einbauten kommen alle üblichen in Destillationskolonnen einsetzbaren Einbeuten in Frage. Bevorzugt werden Einbauten, die einen geringen Druckverlust aufweisen wie Füllkörper, strukturierte Packungen oder Dual-Flow-Böden. Besonders bevorzugt werden strukturierte Packungen.

Der leichtsiedende Strom (5) enthält, wenn der Rohisocyanatstrom (1) durch Phosgenierung in einem leichter als das Isocyanat siedenen Lösemittel erzeugt wurde, u.a. Spuren des Lösungsmittels und/oder chlorhaltige Nebenkomponten. Wenn ein aliphatisches Isocyanat hergestellt wird, enthält der leichtsiedende Strom bevorzugt als chlorhaltige Nebenkomponente eine Verbindung, bei der mindestens Isocyanatgruppe des gewünschten Isocyanates durch Chlor ersetzt ist. Beispielsweise ergeben sich bei der Herstellung von 1,6-Diisocyanatohexan die beiden chlorhaltigen Nebenkomponenten 1-Isocyanato-6-Chlor-Hexan oder 1,6-Dichlorhexan.

Der schwersiedende Strom enthält, wenn der Rohisocyanatstrom durch Phosgenierung erzeugt wurde, chlorhaltige Nebenkomponenten und dimere Nebenprodukte (beispielsweise Carbodiimide und Uretdion).

Bei Anwendung des erfindungsgemäßen Verfahrens ist das Reinisocyanat in einer Reinheit von in der Regel > 99,4, bevorzugt > 99,5 Gew.-% erhältlich.

Als leichtsiedende Hauptverunreinigungen liegen in der Regel Lösungsmittel sowie Nebenprodukte der Phosgenierung vor, wie beispielsweise Methyl-phenyl-isocyanat beim TDI, Phenyl-isocyanat beim MDI und 6-Chlorhexylisocyanat und 1,6-Dichlorhexan beim HDI. Ihr Gehalt beträgt in Reinprodukt typischerweise < 0,5%, bevorzugt < 0,3%. Als hochsiedende Verunreinigungen treten vor allem chlorhaltige Nebenkomponenten auf. Der Gehalt an schwerer als das Isocyanat siendenden chlorhaltigen Nebenkomponenten beträgt bei Ausübung der Erfindung typischerweise <500 ppm, bevorzugt < 100 ppm.

Es sollen nun zwei bevorzugte Ausführungsformen der Erfindung dargestellt werden:

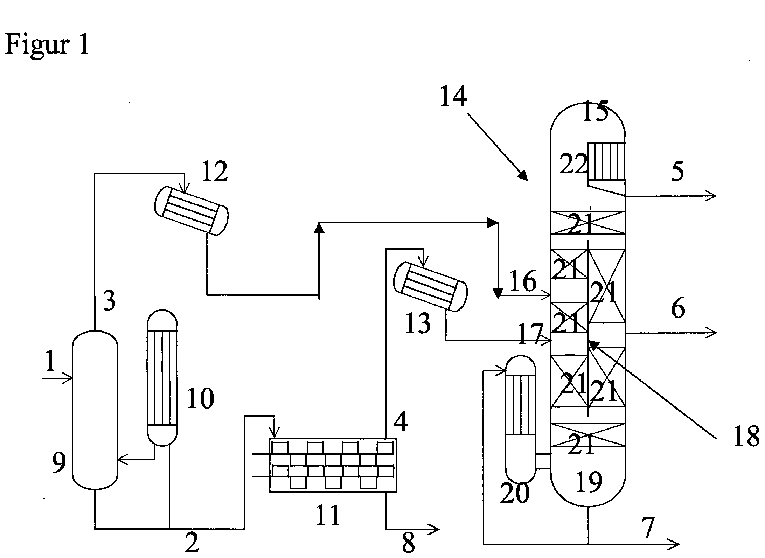

Figur 1 zeigt die erste Ausführung: Der Rohisocyanatstrom (1) wird zur Ausführung des Verfahrensschrittes a) einer einstufigen Verdampfung zugeführt, die aus einem Behälter (9) und einem Verdampfer (10) besteht und im Umlauf betrieben wird. Aus dem Behälter (9) wird der Teilstrom (2) abgezogen, der den nicht verdampfbaren Rückstand enthält. Am Kopf des Behälters (9) wird der Reststrom (3) gasförmig abgezogen.
Zur Ausführung des Verfahrensschrittes c) wird der Teilstrom (2) zur Erzeugung des Brüdenstromes (4) in einer besonders bevorzugten Ausführung einem Schaufeltrockner (11) zugeführt. Aus dem Schaufeltrockner (11) wird der Rückstandstrom (8) nach unten ausgetragen, der im wesentlichen aus nicht verdampfbarem Rückstand besteht. Der gasförmig am Schaufeltrockner abgenommene Strom (4) besteht vor allem aus Isocyanat.

Die beiden Ströme (3) bzw. (4) können in den Kondensatoren (12) bzw. (13) kondensiert werden und einer Trennwandkolonne (14), umfassend Verdampfer (20), Kondensator (22) und trennwirksame Einbauten (21), über die Zuläufe (16) und (17) zugeführt werden. In einer besonders bevorzugten Ausführungsform wird der Strom (3) bzw. dessen Kondensat (16) oberhalb des Stromes (4) bzw. dessen Kondensat (17) zugeführt. Am Kopf (15) der Trennwandkolonne (14) wird der Leichtsiederstrom (5), auf der dem Zulauf gegenüberliegenden Seite der Trennwand (18) wird der Reinisocyanatstrom (6) und am Sumpf (19) wird der Hochsiederstrom (7) abgenommen.

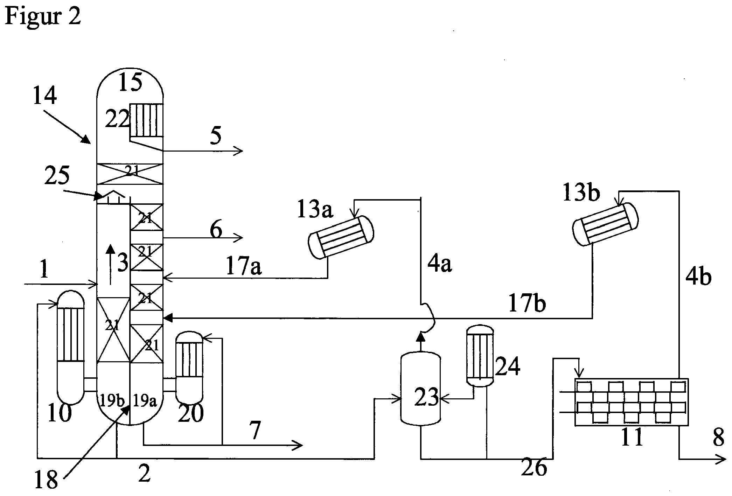

In der zweiten bevorzugten Ausführungsform, die in der Figur 2 gezeigt ist, wird der Rohisocyanatstrom (1) zunächst dem linken Zulaufraum einer Trennwandkolonne (14) mit trennwirksamen Einbauten (21) zugeführt. Die Trennwandkolonne (14) ist so gestaltet, dass die Trennwand (18) bis auf den Boden durchgezogen ist, so dass sich zwei getrennte Sümpfe (19a) und (19b) ergeben. Weiter ist die Trennwandkolonne (14) so gestaltet, dass kein Kondensatstrom vom Kondensator (22) der Trennwandkolonne (14) in den linken Zulaufraum für den Rohisocyanatstrom (1) laufen kann.

Der Trennvorgang im Zulaufraum der Trennwandkolonne (14) mit trennwirksamen Einbauten (21) entspricht einer reinen Abtriebsdestillationsschaltung (d.h. oberhalb des Zulaufs (1) befinden sich im linken Zulaufraum keine trennwirksamen Böden), bei der der abwärtsfallende Flüssigkeitsstrom alleine aus dem Zulauf des Rohisoscyanatstromes (1) besteht. Der Sumpf des Zulaufraumes (19 b) wird im Umlauf mit dem linken Verdampfer (10) betrieben. Aus dem Sumpf (19b) wird Teilstrom (2) mit dem nichtverdampfbaren Rückstand abgezogen.

Durch einen Gashut (25) tritt der Brüdenstrom (3) aus dem linken Zulaufraum in den übrigen Raum der Trennwandkolonne über, ohne daß Kondensat in den linken Zulaufraum gelangt.

Der den nicht verdampfbaren Rückstand enthaltende Teilstrom (2) wird dann dem Verfahrenschritt c) zugeführt. Dort wird zunächst in einer einstufigen Verdampfung, die einen Behälter (23) und einen Verdampfer (24) umfaßt, die im Umlauf betrieben werden, ein erster überwiegend isocyanathaltige Brüdenstrom (4a) erzeugt. Der Austragsstrom (26) aus dem Behälter (23) dieser Anordnung wird einem Schaufeltrockner (11) zugeführt, an dessem Kopf ein zweiter überwiegend isocyanathaltiger Brüdenstrom (4b) anfällt. Nach unten wird aus dem Schaufeltrockner der überwiegend nicht verdampfbaren Rückstand enthaltende Strom (8) ausgeschleust.

Die beiden isocyanathaltigen Ströme (4a) und (4b) werden kondensiert und der Trennwandkolonne (14) zugeführt.

Bei dieser Ausführungsform enthält der Brüdenstrom (3) bereits einen Großteil der leichtsiedenden Komponenten und der Strom (2), sowie die Teilströme (4a) und (4b) sind im wesentlichen frei von Leichsiedern. Die Ströme (3), (4a) und (4b) werden zur Durchführung des Verfahrensschrittes (d) in den übrigen Raum der Trennwandkolonne, der nicht vom linken Zulaufraum eingenommen wird, eingeleitet. Dieser Zulaufraum ist hier gleichzusetzen mit den Raum unterhalb des Bodens mit dem Gashutes (25) auf der linken Seite der Trennwandkolonne (14). Dabei erfolgt besonders bevorzugt die gasförmige Einleitung des Brüdenstromes (3) über den Gashut (25) oberhalb des Abzugs des Reinisocyanatstromes (6) und die Zuführung des flüssigen, isocyanathaltigen Teilstromes (4a) unterhalb des Abzugs des Reinisocyanatstromes (6) und die Einleitung des flüssigen isocyanathaltigen Stromes (4b) unterhalb der Einleitung des flüssigen, isocyanathaltigen Teilstromes (4a).

Am Kopf (15) der Trennwandkolonne wird der Leichsiederstrom (5) und am rechten Sumpf (19a) der Kolonne der Hochsiederstrom (7) abgezogen.

Beiden bevorzugten Ausführungsformen ist das Merkmal b) eigen, dass der Reststrom (3) oder Ströme, die den Reststrom (3) zumindestens teilweise enthalten, vom nicht verdampfbaren Rückstand, der im Teilstrom (2) enthalten ist, getrennt geführt werden. Dies wird in der zweiten bevorzugten Ausführungsform dadurch erreicht, dass die Trennwand (18) der Trennwandkolonne (14) bis auf den Boden durchgezogen wird und dass oberhalb des Zulaufs des Stromes (1) zur Trennwandkolonne (14) kein Kondensatstrom, der durch den Kondensator (22) der Trennwandkolonne (14) erzeugt wird, vorhanden ist, der den brüdenförmigen Reststrom (3) zumindestens teilweise enhalten würde. Der Boden mit dem Gashut (25), über den der brüdenförmige Reststrom (3) in den Bereich oberhalb der Trennwand der Trennwandkolonne gebracht wird, und der den Zulaufraum der Trennwandkolonne vom restlichen Teil der Trennwandkolonne abtrennt, ist erfindungsgemäß konstruktiv so gestaltet, dass der durch den Kondensator (22) erzeugte Kondensatstrom nicht in den Zulaufraum der Trennwandkolonne (14) laufen kann, beispielsweise als Fangboden.

In dieser Schrift verwendete ppm- und Prozentangaben beziehen sich, falls nicht anders angegeben, auf Gewichtsprozente und -ppm.

### Beispiel:

Es wurde der aus einer Phosgenierung von Toluylendiamin (TDA) stammende, Toluylendiisocyanat (TDI) enthaltende Rohisocyanatstrom nach destillativer Abtrennung von HCl, Phosgen und Lösemittel gemäß der in Figur 2 dargestellten bevorzugten Ausführungsform des erfindungsgemäßen Verfahrens zur Herstellung eines Rein-TDI-Stromes eingesetzt. Dabei ergaben sich die folgenden Bedingungen und Mengenströme:

| Strom Nr. | Menge | Zusammensetzung |
|---|---|---|
| 1 | 1 kg/h | 95% TDI; 5% Rückstand (nicht verdampfbar); 170 ppm Leichtsieder; 700 ppm höhersiedende Komponenten |
| 5 | 30 g/h | 99,4% TDI; 0,5% Leichtsieder |
| 6 | 0,92 kg/h | 99,9% TDI; 10 ppm Leichtsieder;50 ppm höhersiedende Komponenten |
| 7 | 1 g/h | 30% TDI; 70% höhersiedende Komponenten |
| 8 | 48 g/h | 98,5% Rückstand; 1,5 % TDI |

| Apparat-Nr. | Bedingungen |
|---|---|
| 14 | Kopfdruck: 18 mbar |
| | Sumpftemperatur (links) 19b: 145 °C |
| | Sumpftemperatur (rechts) 19a: 160 °C |
| | Trennstufen (Bettnummerierung von oben nach unten) |
| | Bett 1: 4 Trennstufen |
| | Zulaufteil: |
| | Bett 2: 2 Trennstufen |
| | Ablaufteil: |
| | Bett 2: 2 Trennstufen |
| | Bett 3: 3 Trennstufen |
| | Bett 4: 2 Trennstufen |
| | Bett 5: 18 Trennstufen |
| 23 | Druck: 8 mbar |
| | Temperatur: 128 °C |
| 11 | Druck: 25 mbar |
| | Temperatur: 240 °C |

## Patentansprüche

1. Verfahren zur Reinigung eines Produktstroms aus der Isocyanatsynthese, umfassend
- destillative Reinigung eines Rohisocyanatstromes (1) im Vakuum bei 1 bis 120 mbar und bei Temperaturen von 90 bis 170 °C in einen gasförmigen Brüden (3) und einen flüssigen Teilstrom (2),
- den nicht verdampfbaren Rückstand im Teilstrom (2) von dem Brüdenstrom (3) und/ oder von Stoffströmen, die den Brüdenstrom (3) zumindest teilweise enthalten, getrennt halten,
- Abtrennung von im flüssigen Teilstrom (2) enthaltenem Isocyanat von darin enthaltenem, nicht verdampfbaren Rückstand als isocyanathaltiger dampfförmiger Reststrom (4),
- destillatives Auftrennen des isocyanathaltigen dampfförmigen Reststroms (4) und des Brüdenstroms (3) in drei Einzelströme (5,6,7) mit unterschiedlichen Siedebereichen, wobei der am leichtesten siedende Strom (5) einen wesentlichen Teil des Leichtsiederanteils des Rohisocyanatstroms (1) enthält, der am schwersten siedende Strom (7) einen wesentlichen Teil des Hochsiederanteils des Rohisocyanatstroms (1) enthält, und der mittelsiedende Strom (6) im Wesentlichen Wertprodukt enthält,
**dadurch gekennzeichnet, daß** man die Abtrennung in einem Schaufeltrockner ohne Kühlzone und mit Austragsschnecke für den nichtverdampfbaren Rückstand (8) durchführt.

2. Verfahren nach Anspruch 1, **dadurch gekennzeichnet, daß** der Rohisocyanatstrom (1) durch Phosgenierung eines Amins, durch Phosgenierung eines Aminhydrochlorids, durch Phosgenierung eines Carbamatsalzes, nach dem Harnstoffverfahren oder durch Umsetzung des zugrundeliegenden Amins mit Dialkylcarbonaten erhalten wurde.

3. Verfahren nach Anspruch 1, **dadurch gekennzeichnet, daß** der Rohisocyanatstrom (1) durch Phosgenierung eines Amins, durch Phosgenierung eines Aminhydrochlorids oder durch Phosgenierung eines Carbamatsalzes erhalten wurde.

4. Verfahren nach Anspruch 1, **dadurch gekennzeichnet, daß** der Rohisocyanatstrom (1) durch Phosgenierung eines Amins erhalten wurde.

5. Verfahren nach einem der Ansprüche 1 bis 3, **dadurch gekennzeichnet, daß** das Isocyanat ausgewählt ist aus der Gruppe bestehend aus monomerem Methylendi(phenylisocyanat (MDI), Toluylendiisocyanat (TDI), R,S-1-Phenylethylisocyanat, 1-Methyl-3-phenylpropylisocyanat, Naphtyldiisocyanat (NDI), n-Pentylisocyanat, 6-Methyl-2-heptanisocyanat, Cyclopentylisocyanat, Hexamethylendiisocyanat (HDI), Isophorondiisocyanat (IPDI), 2,4- und 2,6-Di-isocyanato-methyl-cyclohexan (H₆TDI) und deren Isomerengemische, o-, m- oder p-Xylendiisocyanat (XDI), Di-isocyanato-cyclohexan (t-CHDI), Di-(isocyanato-cyclohexyl)-methan (H₁₂MDI), Tetramethyl-m-xylylendi-isocyanat (m-TMXDI), 1,3-Bis(isocyanatomethyl)cyclohexan (H₆XDI), 1,6-Di-isocyanato-2,2,4,4-tetramethylhexan, 1,6-Di-isocyanato-2,2,4-trimethylhexan und deren Gemische (TMDI).

6. Verfahren nach einem der Ansprüche 1 bis 3, **dadurch gekennzeichnet, daß** das Isocyanat ausgewählt ist aus der Gruppe bestehend aus monomerem Methylendi(phenylisocyanat (MDI), Toluylendiisocyanat (TDI), Naphtyldiisocyanat (NDI), Hexamethylendiisocyanat (HDI), Isophorondiisocyanat (IPDI), 2,4- und 2,6-Di-isocyanato-methyl-cyclohexan (H₆TDI) und deren Isomerengemische, o-, m- oder p-Xylendiisocyanat (XDI), Di-(isocyanato-cyclohexyl)-methan (H₁₂MDI) und Di-isocyanato-cyclohexan (t-CHDI).

7. Verfahren nach einem der vorstehenden Ansprüche, **dadurch gekennzeichnet, daß** man die Abtrennung bei 80 bis 320 °C durchführt.

8. Verfahren nach einem der vorstehenden Ansprüche, **dadurch gekennzeichnet, daß** man die Abtrennung bei 0,1 bis 40 mbar durchführt.

## Claims

1. A process for purifying a product stream from isocyanate synthesis, comprising
- purification of a crude isocyanate stream (1) by distillation under reduced pressure at from 1 to 120 mbar and at temperatures of from 90 to 170°C to give a gaseous vapor (3) and a liquid substream (2),
- keeping the unvaporizable residue in the part-stream (2) separate from the vapor stream (3) and/or from streams which at least partly comprise the vapor stream (3),
- separation of isocyanate comprised in the liquid substream (2) from nonvaporizable residue comprised therein as isocynate-comprising gaseous residual stream (4),
- separation of the isocyanate-containing vapor stream (4) and the vapor stream (3) into three individual streams (5, 6, 7) having different boiling ranges by distillation, the lowest-boiling stream (5) comprising a substantial part of the low boiler content of the crude isocyanate stream (1), the highest-boiling stream (7) comprising a substantial part of the high boiler contend of the crude isocyanate stream and the medium-boiling stream (6) substantially comprising desired product,
wherein the separation is carried out in a paddle drier without cooling zone and with discharge screw for the nonvaporizable residue (8).

2. The process according to claim 1, wherein the crude isocyanate stream (1) has been obtained by phosgenation of an amine, by phosgenation of an amine hydrochloride, by phosgenation of a carbamate salt, by the urea process or by reaction of the parent amine with dialkyl carbonates.

3. The process according to claim 1, wherein the crude isocyanate stream (1) has been obtained by phosgenation of an amine, by phosgenation of an amine hydrochloride or by phosgenation of a carbamate salt.

4. The process according to claim 1, wherein the crude isocyanate stream (1) has been obtained by phosgenation of an amine.

5. The process according to any of claims 1 to 3, wherein the isocyanate is selected from the group consisting of monomeric methylenedi(phenyl isocyanate) (MDI), tolylene diisocyanate (TDI), R,S 1 phenylethyl isocyanate, 1 methyl-3-phenyl-propyl isocyanate, naphthyl diisocyanate (NDI), n pentyl isocyanate, 6 methyl-2-heptane isocyanate, cyclopentyl isocyanate, hexamethylene diisocyanate (HDI), isophorone diisocyanate (IP-DI), 2,4 and 2,6 diisocyanatomethylcyclohexane (H6TDI) and isomer mixtures thereof, o-, m- or p xylene diisocyanate (XDI), diisocyanatocyclo¬hexane (t CHDI), di(isocyanatocyclohexly)methane (H12MDI), tetramethyl-m-xylylene diisocyanate (m TMXDI), 1,3 bis(isocyanatomethyl)cyclohexane (H6XDI), 1,6 diisocyanato-2,2,4,4-tetramethyl-hexane, 1,6 diisocyanato-2,2,4-trimethylhexane and mixtures thereof (TM-DI).

6. The process according to any of claims 1 to 3, wherein the isocyanate is selected from the group consisting of monomeric methylenedi(phenyl isocyanate) (MDI), tolylene diisocyanate (TDI) , naphthyl diisocyanate (NDI), hexamethylene diisocyanate (HDI), isophorone diisocyanate (IPDI), 2,4- and 2,6 diisocyanatomethylcyclohexane (H6TDI) and isomer mixtures thereof, o-,m- or p xylene diisocyanate (XDI), di(isocyanatocyclohexly)methane (H12MDI) and diisocyanato-cyclohexane (t CHDI).

7. The process according to any of the preceding claims, wherein the separation is carried out at from 80 to 320°C.

8. The process according to any of the preceding claims, wherein the separation is carried out at from 0.1 to 40 mbar.

## Revendications

1. Procédé de purification d'un courant de produits issu de la synthèse d'un isocyanate, comprenant
- la purification par distillation d'un courant d'isocyanate brut (1) sous vide à 1 à 120 mbar et à des temperatures de 90 à 170 °C en une vapeur gazeuse (3) et un courant partiel liquide (2),
- le maintien séparé du résidu non évaporable dans le flux partiel (2) du flux de vapeur (3) et/ou de flux de matières qui comprennent au moins partiellement le flux de vapeur (3),
- la séparation de l'isocyanate contenu dans le courant partiel liquide (2) du résidu non évaporable qu'il contient en tant que courant résiduel gazeux contenant l'isocyanate (4),
- la séparation par distillation du ou des flux de vapeur (4) contenant l'isocyanate et le flux de vapeur (3) en trois flux individuels (5, 6, 7) avec des domaines d'ébullition differérents, le flux (5) au point d'ébullition le plus bas contenant une partie essentielle de la partie du corps à bas point d'ébullition du flux d'isocyanate brut (1), le flux au point d'ébullition le plus élevé (7) contenant une partie essentielle de la partie du corps à haut point d'ébullition du flux d'isocyanate brut (1) et le flux à point d'ébullition moyen (6) contenant essentiellement du produit de valeur,
**caractérisé en ce que** la séparation est réalisée dans un séchoir à pale sans zone de refroidissement et muni d'une vis de déchargement pour le résidu non évaporable (8).

2. Procédé selon la revendication 1, **caractérisé en ce que** le courant d'isocyanate brut (1) a été obtenu par phosgénation d'une amine, par phosgénation d'un chlorhydrate d'amine, par phosgénation d'un sel de carbamate, par le procédé à l'urée ou par mise en réaction de l'amine sous-jacente avec des carbonates de dialkyle.

3. Procédé selon la revendication 1, **caractérisé en ce que** le courant d'isocyanate brut (1) a été obtenu par phosgénation d'une amine, par phosgénation d'un chlorhydrate d'amine ou par phosgénation d'un sel de carbamate.

4. Procédé selon la revendication 1, **caractérisé en ce que** le courant d'isocyanate brut (1) a été obtenu par phosgénation d'une amine.

5. Procédé selon l'une quelconque des revendications 1 à 3, **caractérisé en ce que** l'isocyanate est choisi dans le groupe constitué par le di(phénylisocyanate) de méthylène (MDI), le diisocyanate de toluylène (TDI), l'isocyanate de R,S-1-phényléthyle, l'isocyanate de 1-méthyl-3-phénylpropyle, le diisocyanate de naphtyle (NDI), l'isocyanate de n-pentyle, l'isocyanate de 6-méthyl-2-heptane, l'isocyanate de cyclopentyle, le diisocyanate d'hexaméthylène (HDI), le diisocyanate d'isophorone (IPDI), le 2,4- et le 2,6-di-isocyanato-méthyl-cyclohexane (H6TDI) et leurs mélanges d'isomères, le diisocyanate d'o-, m- ou p-xylène (XDI), le di-isocyanato-cyclohexane (t-CHDI), le di-(isocyanato-cyclohexyl)-méthane (H12MDI), le diisocyanate de tétraméthylm-xylylène (m-TMXDI), le 1,3-bis(isocyanatométhyl)cyclohexane (H6XDI), le 1,6-diisocyanato-2,2,4,4-tétraméthylhexane, le 1,6-diisocyanato-2,2,4-triméthylhexane et ses mélanges (TMDI).

6. Procédé selon l'une quelconque des revendications 1 à 3, **caractérisé en ce que** l'isocyanate est choisi dans le groupe constitué par le di(phénylisocyanate) de méthylène (MDI), le diisocyanate de toluylène (TDI), le diisocyanate de naphtyle (NDI), le diisocyanate d'hexaméthylène (HDI), le diisocyanate d'isophorone (IPDI), le 2,4- et 2,6-diisocyanato-méthyl-cyclohexane (H6TDI) et leurs mélanges d'isomères, le diisocyanate d'o-, m- ou p-xylène (XDI), le di-(isocyanato-cyclohexyl)-méthane (H12MDI) et le diisocyanato-cyclohexane (t-CHDI).

7. Procédé selon l'une quelconque des revendications précédentes, **caractérisé en ce que** la séparation est réalisée à 80 à 320 °C.

8. Procédé selon l'une quelconque des revendications précédentes, **caractérisé en ce que** la séparation est réalisée à 0,1 à 40 mbar.
